# FASCICULE DE BREVET EUROPEEN

(11) **EP 1 765 227 B1**
(45) Date de publication et mention de la délivrance du brevet: **02.07.2014**
(21) Numéro de dépôt: 05752647.7
(22) Date de dépôt: 31.05.2005
(51) Int. Cl.: A61F 2/38

(54) **PROTHESE TOTALE DU GENOU**
KOMPLETTE KNIEPROTHESE
COMPLETE KNEE PROSTHESIS

(30) Priorité: 17.06.2004 FR 0406556
(43) Date de publication de la demande: 28.03.2007
(73) Titulaire: Transysteme - JMT Implants, 30000 Nimes (FR); Couette, Philippe, 75007 Paris (FR)
(72) Inventeur: COUETTE, Philippe, F-75007 Paris (FR)
(74) Mandataire: Cornuejols, Georges
(86) Numéro de dépôt international: PCT/EP2005/052490
(87) Numéro de publication internationale: WO 2005/122967

(56) Documents cités:
- EP-A- 0 993 812
- US-A- 4 888 021
- US-A- 5 725 584
- US-A- 6 013 103

## Description

La présente invention est du domaine des techniques médicales et plus particulièrement des prothèses des articulations. Elle a pour objet une prothèse totale du genou.

On rappelle qu'une prothèse totale du genou associe trois éléments, dont un élément fémoral et un élément tibial destinés à être ancrés respectivement dans le fémur et le tibia, et un élément intermédiaire interposé entre eux. L'élément tibial est conformé en plateau équipé d'une quille axiale destinée à être insérée à l'intérieur d'un logement ménagé dans le tibia, de manière à prendre appui contre l'extrémité de ce dernier préalablement préparée par retrait de matière osseuse. Cet élément tibial comporte lui-même un logement axial de réception d'une quille épaulée de l'élément intermédiaire, pour leur jonction l'un à l'autre en appui axial de l'élément intermédiaire contre l'élément tibial. L'élément intermédiaire comporte à la face opposée de celle d'appui axial contre l'élément tibial, un logement composé d'un couple de cavités à portée globalement sphérique, pour la réception de condyles respectifs droit et gauche de l'élément fémoral. Ces cavités de l'élément intermédiaire sont séparées par un bossage médian favorisant l'appui de l'élément fémoral, qui coopère avec une échancrure de séparation des condyles l'un de l'autre. L'élément fémoral, outre les condyles et l'échancrure, comporte à la face opposée de ces derniers une double concavité et un bossage, à l'instar de ceux de l'élément intermédiaire, pour loger en correspondance les condyles et l'échancrure naturels du fémur préalablement préparés, auquel fémur l'élément fémoral est fixé.

La difficulté majeure à surmonter dans le domaine réside dans la reproduction la plus fidèle possible par la prothèse des conditions de mobilité anatomique du genou, y compris en prenant en compte une perte fréquente des ligaments croisés. De telles conditions de mobilité doivent être obtenues sans perte de congruence, en optimisant l'importance et la constance des surfaces d'appui de l'élément fémoral sur l'élément intermédiaire.

Si les tensions ligamentaires doivent être maintenues pour autoriser en permanence le mouvement, celles-ci ne doivent néanmoins pas induire un surcroît inopportun et inadapté de ces tensions, qui sont susceptibles de provoquer une fatigue et des douleurs pour le patient. Il en ressort qu'une organisation générale inadéquate de la prothèse peut induire chez le patient des efforts inopportuns à fournir, pour notamment compenser une prise d'appui inadaptée de l'élément fémoral sur l'élément intermédiaire au cours du passage entre les stations d'extension et les différentes stations successives de flexion du genou.

En outre, cette mobilité doit être préférentiellement obtenue en préservant le capital osseux et en limitant les résections osseuses nécessaires à l'implantation de la prothèse. Cette difficulté est rendue d'autant plus délicate à surmonter, que les prothèses sont élaborées pour être implantées sur de quelconques patients susceptibles de présenter une conformation d'articulation du genou qui leur est propre. Si l'utilisation courante d'une gamme de prothèses de même conformation à des dimensions diverses réparties par paliers dimensionnels permet de surmonter partiellement cette difficulté, elle n'en facilite pas moins les efforts que doivent développer les concepteurs pour organiser une prothèse transposable à chacun de ces paliers. Il en ressort pour les concepteurs du domaine la recherche d'un compromis entre les contraintes dimensionnelles de la prothèse, d'une taille à l'autre, et celles liées à sa mobilité.

Plus précisément, la mobilité offerte par la prothèse doit permettre une rotation en flexion et en extension dans le plan sagittal, autour d'un axe horizontal, de l'élément fémoral sur l'élément intermédiaire. Cette rotation doit être la plus proche possible des conditions anatomiques normales, et doit non seulement prendre en compte des contraintes cinématiques de mobilité générale de l'élément fémoral par rapport à l'élément tibial, mais aussi des contraintes relatives à la stabilité que doit offrir la prothèse en de quelconques positions de flexion et/ou d'extension.

Il en ressort que la congruence et les surfaces d'appui de l'élément fémoral sur l'élément intermédiaire doivent être optimisées, surtout pour des positions de la prothèse dans lesquelles les charges d'appui qu'exerce le patient sont les plus importantes. Plus précisément, ces positions d'appui correspondent à différentes stations de flexion intermédiaires, telles que des stations de flexion du patient lorsqu'il progresse sur un escaller. En outre, une telle stabilité de la prothèse, tant antéro-postérieure que rotatoire, doit être offerte en prenant en compte une rotation dans le plan horizontal entre l'élément intermédiaire et l'élément tibial, tout en autorisant, , sans perte de congruence, un basculement d'un condyle à l'autre autour d'un axe horizontal antéro-postérieur. On relèvera que ces diverses mobilités des éléments composant la prothèse entre eux, relèvent encore de choix et de recherche de compromis auxquels sont soumis les concepteurs.

Il est notamment connu d'aménager à cet effet des moyens médians spécifiques de stabilisation latérale de l'élément fémoral par rapport à l'élément intermédiaire. La rotation de l'élément fémoral sur l'élément intermédiaire doit être autorisée sans pour autant induire un risque d'échappée des condyles au-delà de l'élément intermédiaire, susceptible de provoquer une luxation: Il a été proposé de prolonger vers l'avant le bossage de l'élément intermédiaire par une lèvre de retenue. Cependant, la proéminence de cette lèvre tend à induire une gêne pour le patient et à accroître inconsidérément la taille de l'élément intermédiaire. Une telle solution implique de rendre la proéminence de la lèvre la plus faible possible, avec en corollaire une Influence sur l'organisation de la mobilité de l'élément fémoral sur l'élément intermédiaire et de leur conformation complémentaire. Inversement, les facultés de mobilité offertes à la flexion ne doivent néanmoins pas porter atteinte à l'obtention d'une position idoine d'extension, et l'organisation de la portée des condyles de l'élément fémoral sur les cavités de l'élément intermédiaire doit prendre en compte le passage de l'une à l'autre de ces positions dans les meilleures conditions de stabilité susvisées.

Le Brevet US 6 013 103 (KAUFMAN et al.) décrit une prothèse de genou dans laquelle l'élément intermédiaire est monté glissant dans un plan horizontal sur l'élément tibial. Le guidage est réalisé par des rails bordant l'élément intermédiaire entre lesquels un pion est reçu. Un tel montage n'autorise pas un pivotement de l'élément intermédiaire sur l'élément tibial.

Il en ressort qu'il est souhaitable que la portée des condyles de l'élément fémoral à l'intérieur de l'élément intermédiaire, organisée en zone postérieure de l'élément fémoral, s'étende vers la zone antérieure de ce dernier avec des rayons de courbure et des zones d'inflexion dont les caractéristiques sont élaborées en fonction des choix, des contraintes et des compromis à rechercher susvisés, au regard d'agencement et de structures spécifiques des éléments étagés qui composent la prothèse.

Pour connaître un environnement technologique proche de la présente invention, on pourra par exemple se reporter aux documents EP1354571 (SCORE PTG), WO0106961 (BERCOVY), et WO03068109 (AFRIAT) qui décrivent de telles prothèses et en exposent la problématique.

Il a aussi été proposé par US5725584 (WALKER et al.) d'organiser une prothèse de genou en un élément fémoral circulant en rotation sur un élément tibial. Pour éviter une perte de congruence lors du mouvement de flexion, le rayon de l'élément fémoral est un rayon constant. Cependant, une telle solution présente des inconvénients. En premier lieu, les efforts de torsion et de cisaillement sont transmis de l'un à l'autre des éléments de la prothèse. En second lieu, la liberté de mouvement offerte pour une mobilité satisfaisante de la prothèse, et le mouvement de basculement limitant les tensions ligamentaires ne sont pas obtenus. En troisième lieu, le capital osseux n'est pas préservé, une résection osseuse importante étant requise en bout distal antérieur du fémur. En quatrième lieu, le bras de levier de la rotule est réduit à une courte distance en cours de flexion. Pour surmonter au moins en partie ces inconvénients, il est plus particulièrement proposé de faire varier la courbure de la partie antérieure de l'élément fémoral. La zone de guidage à rayon constant s'étend sur une plage de l'ordre de 90°, en étant latéralement ménagée sur les condyles de l'élément fémoral, entre lesquels est ménagé un évidement coopérant avec un bossage ménagé sur l'élément tibial, de manière à former une cage inter condylienne destinée à recevoir un dispositif de postéro stabilisation.

Il a aussi été proposé par US4888021 (FORTE et al.) une prothèse du genou composée d'un élément fémoral, d'un élément tibial et d'un élément intermédiaire interposé entre eux, à la manière de l'art antérieur précédemment décrit. L'élément fémoral est relié en rotation à l'élément intermédiaire au moyen d'une pièce annexe portée par l'élément intermédiaire et d'un tourillon. De telles dispositions impliquent une perte du capital osseux du fémur, ne permettent pas un basculement d'un condyle à l'autre pour réduire les tensions ligamentaires du patient, notamment en charge, et n'offrent pas les conditions souhaitées requises d'une congruence totale quelle que soit la position de la prothèse.

La présente invention s'inscrit dans le cadre des contraintes qui ont été exposées, et vise à proposer une prothèse totale du genou offrant un confort satisfaisant pour le patient.

La prothèse de la présente invention est une prothèse totale du genou composée d'un élément tibial à plateau, d'un élément fémoral et d'un élément intermédiaire interposé entre les précédents. L'élément tibial est pourvu de moyens d'ancrage et de centrage à l'extrémité d'un tibia, et de moyens de réception d'un épaulement et d'une quille d'emboîtement axial et de centrage de l'élément intermédiaire. L'élément fémoral est quant à lui pourvu de moyens d'ancrage et de centrage à l'extrémité d'un fémur, qui sont ménagés sur une face interne de conformation globalement complémentaire à celle des condyles et de l'échancrure naturels du fémur préalablement préparés, de manière à les envelopper. Cette face interne comporte plus particulièrement deux facettes opposées, ou analogues, qui sont distantes l'une de l'autre d'une distance d4. On comprendra qu'à la manière habituelle dans le domaine, cette distance d4 est notamment déterminante quant au choix de la taille de la prothèse à implanter, parmi une gamme dimensionnelle de ces prothèses.

L'élément fémoral présente aussi une face externe comportant quant à elle un couple de condyles séparés par une échancrure, qui sont ménagés sur la partie postérieure de l'élément fémoral pour former une surface de guidage de ce dernier sur l'élément intermédiaire le profil de ces condyles s'étendant suivant un rayon R1 constant. Ce dernier est interposé entre l'élément tibial et l'élément fémoral de manière à autoriser une mobilité de l'élément fémoral sur l'élément intermédiaire. Cette mobilité associe une rotation de l'élément fémoral sur l'élément intermédiaire dans le plan sagittal autour d'un axe horizontal a, un basculement d'un condyle à l'autre de l'élément fémoral. Cet élément intermédiaire comporte à sa face externe un logement composé de cavités séparées l'une de l'autre par un bossage, pour la réception respectivement des condyles et de l'échancrure de l'élément fémoral.

Selon la présente invention, une prothèse du genre susvisée est reconnaissable en ce qu'elle comporte les caractéristiques suivantes, prises seules ou en combinaison.

Selon un premier aspect de la présente invention, l'élément intermédiaire autorise un pivotement axial de lui-même sur l'élément tibial et en considérant le plan sagittal latéralisé relatif à l'un et/ou l'autre des condyles de l'élément fémoral, le profil de ces condyles s'étend suivant un rayon R1 constant de rotation plus important que le rayon anatomique correspondant des condyles d'un fémur. Ces dispositions visent notamment à optimiser les surfaces de contact en préservant le capital osseux du fémur. Ce rayon R1 constant s'étend notamment sur une plage P1 de surface de guidage angulaire correspondante de l'ordre de 150° au moins.

Grâce à la portée de l'élément fémoral suivant un rayon constant plus important que le rayon naturel des condyles d'un fémur, les surfaces d'appui de l'élément fémoral sur l'élément intermédiaire sont augmentées et la concentration des charges sur le bord postérieur de l'élément intermédiaire est évitée. En outre, il est possible d'augmenter la distance entre te bord antérieur de l'élément intermédiaire et le fond des cavités de réception des condyles de l'élément fémoral, ce qui permet d'augmenter la stabilité antéro-postérieure. Par ailleurs, le rayon frontal des condyles est susceptible d'être réduit, ce qui permet d'augmenter la stabilité rotatoire et de diminuer la translation latérale nécessaire au basculement d'un condyle à l'autre, sans perte de congruence. Pendant toute la phase de flexion nécessaire aux mouvements en change, de l'ordre supérieur à 100° de flexion, la congruence totale est maintenue dans le plan frontal et dans le plan sagittal, le rayon R1 de rotation pouvant être maintenu jusqu'à 105°. Au-delà de 105°, la tension sur les ligaments latéraux est réduite pour faciliter la fin de flexion sans induire un inconfort pour le patient.

Ledit rayon R1 est de l'ordre de 0,5 fois la distance d4 de séparation desdites facettes opposées de l'élément fémoral. On relèvera que cette proportion est susceptible d'être pondérée selon la taille de la prothèse, de plus ou moins 15% sans sortir du cadre de la présente invention.

Selon un autre aspect de la présente invention, l'axe a de rotation des condyles sur l'élément intermédiaire est décalé dans le plan sagittal vers l'arrière d'une distance d1 par rapport à l'axe A de pivotement de l'élément intermédiaire sur l'élément tibial. Un tel décalage Induit un moment important d'action en extension de la prothèse, et augmente les possibilités de flexion en empêchant un contact prématuré entre la face postérieure du fémur et le bord postérieur de l'élément tibial. On notera qu'un tel décalage est notamment rendu possible grâce à la dimension susvisée du rayon Bi constant de rotation plus important qde le rayon anatomique correspondant des condyles d'un fémur.

A titre indicatif, ladite distance d1 est de l'ordre de 0,18 fois la distance d4 de séparation desdites facettes opposées de l'élément fémoral. On relèvera que cette proportion est susceptible d'être pondérée selon la taille de la prothèse, de plus ou moins 10% sans sortir du cadre de la présente invention.

On relèvera à ce stade de la description que les dispositions relatives aux aspects susvisés, prises préférentiellement en combinaison, permettent un rehaussement de l'élément intermédiaire par une lèvre de retenue de l'extrémité postérieure de l'élément fémoral, dont l'élévation par rapport à la surface de portée des condyles sur les cavités est susceptible d'être réduite pour éviter une gêne pour le patient.

Plus précisément et selon un autre aspect de la présente invention, le bord antérieur de l'élément intermédiaire étant de préférence rehaussé d'une lèvre, celle-ci est avantageusement ménagée en prolongement de l'une et l'autre des cavités de l'élément intermédiaire, pour conférer en prolongement de l'échancrure une conformation de l'élément fémoral en trochlée anatomique. En outre, un tel agencement de la lèvre permet de ménager une distance d2 de séparation suffisante mais néanmoins limitée, entre le fond des cavités et le bord supérieur de la lèvre.

A titre indicatif ladite distance d2 est de l'ordre de 0,32 fois la distance d4 de séparation desdites facettes opposées de l'élément fémoral. On relèvera que cette proportion est susceptible d'être pondérée selon la taille de la prothèse, de plus ou moins 5% sans sortir du cadre de la présente invention.

Le débattement angulaire de l'élément fémoral sur l'élément intermédiaire entre les stations extrêmes d'extension et de flexion est notamment de l'ordre de 120°. Les surfaces de portée s'étendent sur une plage de surface angulaire de l'ordre de 90°, pour répondre au mieux à un compromis entre une portée sensiblement constante de l'élément fémoral sur l'élément intermédiaire, pour des angles de flexion qui correspondent aux charges les plus importantes que doit supporter la prothèse telle qu'organisée par la présente invention. Plus particulièrement, cette portée est sensiblement constante depuis la station d'extension à 0° de verticalité jusqu'à 90° de flexion. Cette plage angulaire se réduit ensuite préférentiellement fortement jusqu'à 120° de flexion. De telles dispositions, en relation avec l'un quelconque au moins des aspects de l'invention susvisés, permettent notamment de réduire les tensions ligamentaires pour le patient, en station d'efforts à exercer les plus conséquents, sans inversement provoquer inopportunément de telles tensions lorsqu'elles sont inutiles.

Pris dans le plan frontal, le rayon R3 des cavités de l'élément intermédiaire est à titre indicatif de l'ordre de 0,6 fois la distance d4 de séparation desdites facettes opposées de l'élément fémoral, tandis que le rayon R2 du bossage de l'élément intermédiaire est quant à lui de l'ordre de 10 mm. On notera que ces dimensions sont à pondérer selon la taille de la prothèse, d'une valeur de l'ordre de plus ou moins 5% sans sortir du cadre de la présente invention. Ces dispositions sont telles que la conformation du bossage confère une mobilité continue et souple audit basculement de l'un à l'autre des condyles à partir d'un rayon R2 réduit, pour éviter des déplacements inopportuns de l'élément fémoral sur l'élément intermédiaire.

De préférence, la face interne de l'élément fémoral comporte des cavités de réception respective des condyles du fémur, qui sont disposées de part et d'autre d'un bossage correspondant à la face opposée de l'échancrure de l'élément fémoral, ce bossage étant destiné à coopérer avec l'échancrure du fémur.

Ces cavités de réception des condyles du fémur sont avantageusement chacune conformées en un plan médian comportant au moins un picot d'ancrage de l'élément fémoral dans le fémur, constituant lesdits moyens d'ancrage de l'élément fémoral à l'intérieur du fémur préalablement préparé. Ce plan médian est bordé de part et d'autre par des plans inclinés à 45°, respectivement antérieur et postérieur, eux-mêmes respectivement bordés par des plans d'extrémité antérieur et postérieur formant lesdites facettes opposées. Ces plans d'extrémité antérieur et postérieur délimitent ensemble un angle ouvert pour faciliter l'impaction de l'élément fémoral sur le fémur, sans endommager l'os et son revêtement.

On relèvera que la partie postérieure de l'élément fémoral, prise dans le plan sagittal latéralisé relatif à l'un et/ou l'autre des condyles, est préférentiellement prolongée par une partie antérieure de l'élément fémoral à large rayon de courbure, lui conférant un profil sensiblement droit, qui est ensuite fortement recourbée.

Il apparaît que grâce aux dispositions de l'invention, la surface de guidage des condyles de l'élément fémoral sur les cavités de l'élément intermédiaire est plus élevée que la surface naturelle correspondante de la partie postérieure des condyles d'un fémur. La surface effective de portée lors des mouvements de flexion en charge par le patient, est sensiblement maintenue constante sur une plage angulaire de l'ordre de 90°, pour offrir une congruence totale dans le plan frontal et dans le plan sagittal pendant ces mouvements, tandis que cette plage se trouve réduite en fin de flexion pour réduire les tensions sur les ligaments latéraux du patient. Il en ressort qu'une concentration des charges sur le bord postérieur de l'élément intermédiaire est évitée, la distance entre le bord antérieur de l'élément intermédiaire et le fond des cavités étant susceptible d'être étendue pour favoriser la stabilité antéro-postérieure de la prothèse. Le rayon frontal des condyles de l'élément fémoral est quant à lui susceptible d'être réduit, pour favoriser la stabilité en rotation tout en diminuant la translation latérale nécessaire au basculement de l'un à l'autre des condyles, sans perte significative de congruence. En outre, un dispositif central de stabilisation peut être évité, grâce à la congruence obtenue et à la distance de séparation entre le bord antérieur et le fond des cavités de l'élément intermédiaire qui peut être augmentée. La suppression de la cage inter condylienne habituellement destinée à recevoir le dispositif de postéro stabilisation permet de réaliser une trochlée anatomique particulièrement adaptée à la conservation de la rotule naturelle, qui reste congruente tout au long de la flexion.

La présente invention sera mieux comprise, et des détails en relevant apparaîtront, à la lecture de la description qui va en être faite d'une forme préférée de réalisation, en relation avec les figures des planches annexées, dans lesquelles :
Les fig.1 et fig.2 sont des illustrations d'une prothèse totale du genou selon une forme préférée de réalisation de la présente invention, respectivement en perspective et en vue sagittale.
Les fig.3 et fig.4 sont des illustrations en perspective selon des angles de vue interne et externe d'un élément fémoral participant de la prothèse illustrée sur les fig.1 et fig.2.
La fig.5 est une illustration en vue sagittale de l'élément fémoral représenté sur les fig.3 et fig.4.
Les fig.6, fig.7 et fig.8 sont des illustrations d'un élément intermédiaire participant de la prothèse représentée sur les fig.1 et fig.2, respectivement en perspective, en vue frontale et en vue sagittale.
Les fig.9 et fig.10 sont des représentations schématiques d'un élément fémoral d'une prothèse de genou, illustrant la comparaison entre la conformation d'une prothèse de l'invention et une prothèse de l'art antérieur, respectivement en vue sagittale et en vue frontale.

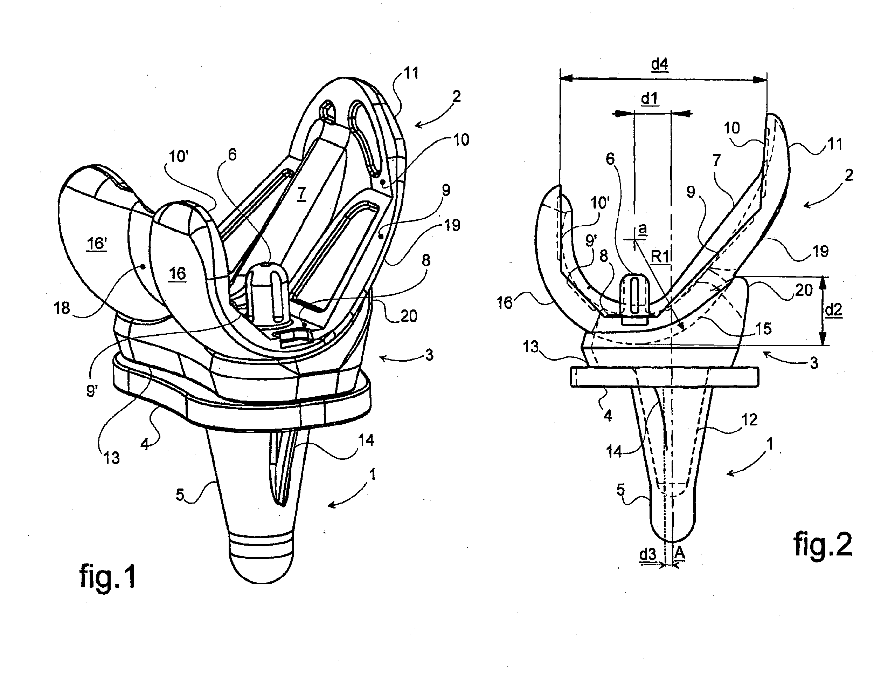

Sur les fig.1 et fig.2, une prothèse totale du genou est principalement composée d'un élément tibial 1, d'un élément fémoral 2, et d'un élément intermédiaire 3 interposé entre eux 1 et 2. L'élément tibial 1 comporte un plateau 4 d'appui à l'extrémité du tibia du patient, et une quille 5 de centrage à l'intérieur d'un dégagement préalablement pratiqué dans ce dernier. Cette quille 5 comporte de préférence des ailes latérales 14 permettant d'immobiliser l'élément tibial 1 en rotation sur lui-même à l'intérieur du tibia du patient.

L'élément fémoral 2 comporte quant à lui à sa face interne des picots, tels que 6, d'ancrage à l'intérieur du fémur du patient, lui aussi préalablement préparé. On notera à ce stade de la description que la préparation préalable du fémur comporte les opérations de retrait de matière osseuse pour recevoir des zones planes correspondantes de la face interne de l'élément fémoral 2, qui sont disposées de part et d'autre d'un bossage 7 destiné à être reçu par l'échancrure naturelle du fémur.

Ces zones planes comprennent des plans médians 8, en saillie desquels sont ménagés les picots 6, ces plans médians 8 étant prolongés par des plans inclinés à 45°, respectivement antérieur 9 et postérieur 9', puis par des plans d'extrémité respectivement antérieur 10 et postérieur 10'. On notera que ces plans d'extrémité antérieur 10 et postérieur 10' sont sensiblement parallèles à un écart angulaire près de l'ordre de 4° pour former conjointement un angle ouvert, le plan d'extrémité antérieur 10 étant ménagé sur une zone 11 recourbée de l'élément fémoral 2. Ces plans d'extrémité antérieur 10 et postérieur 10' forment des facettes de l'élément fémoral 2 dont la distance d4 de séparation est déterminante pour le choix du chirurgien quant à la taille de la prothèse à implanter. Cette distance d4 est notamment comprise entre 40 mm et 58 mm selon la taille de la prothèse.

L'élément intermédiaire 3 comporte quant à lui une quille 12 de centrage à l'intérieur de l'élément tibial 1, pour prendre appui contre la face supérieure du plateau 4 de l'élément tibial 1 par l'intermédiaire d'un épaulement 13 de la quille 12, à l'instar de l'appui que prend l'élément tibial 1 à l'extrémité du tibia.

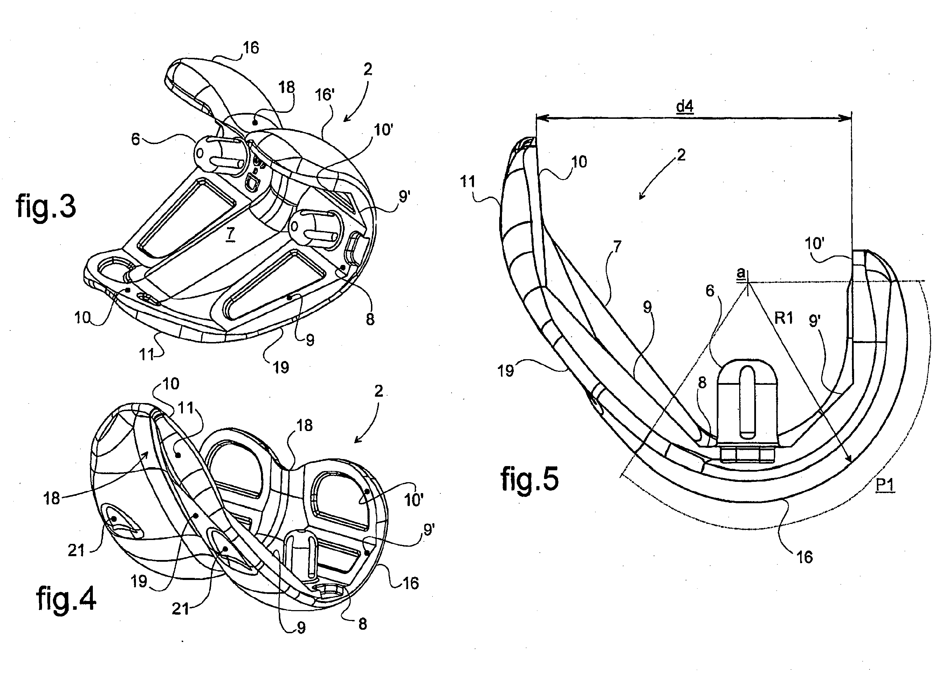

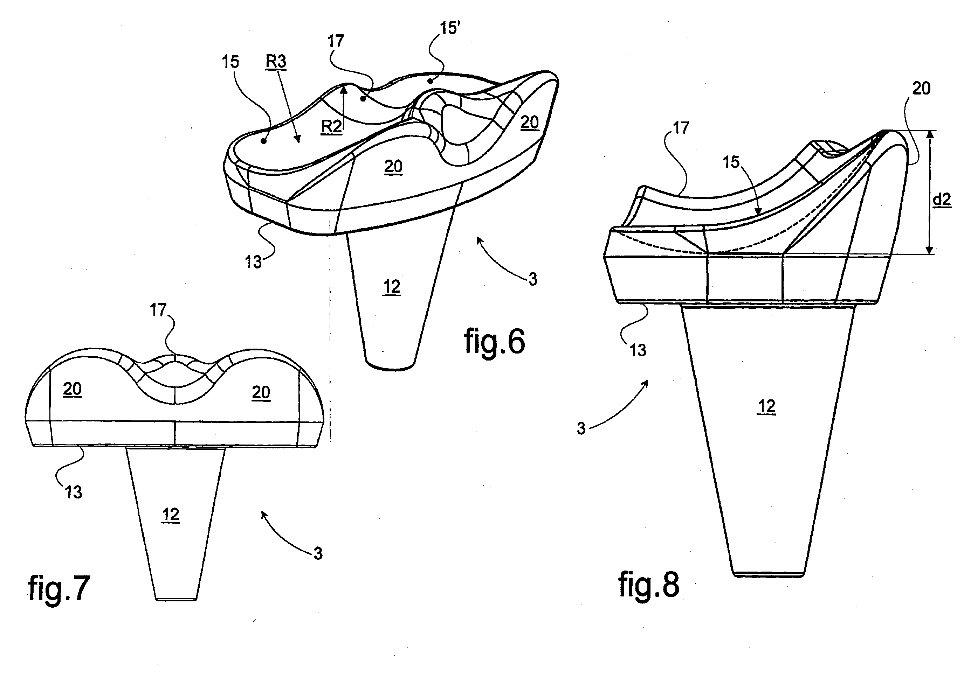

En se reportant aux fig.3 à fig.5 d'une part, et aux fig.7 à fig.8 d'autre part, l'élément fémoral 2 est destiné à prendre appui contre l'élément intermédiaire 3 pour son guidage en mobilité en rotation dans le plan sagittal, autour d'un axe horizontal a. A cet effet, les éléments fémoral 2 et intermédiaire 3 comportent des reliefs complémentaires, dont un couple de cavités concaves 15,15' de l'élément intermédiaire 3 pour la réception en rotation de condyles respectifs 16,16' de l'élément fémoral 2. Les cavités 15 et 15' de l'élément intermédiaire 3 sont séparées l'une de l'autre par un bossage 17 destiné à recevoir une échancrure 18 de l'élément fémoral 2. Sur la fig.6 plus particulièrement, la concavité des cavités 15 et 15' de l'élément intermédiaire 3, prise dans le plan frontal, est d'un rayon R3 de l'ordre de compris entre 24 mm et 32 mm selon la taille de la prothèse, tandis que sur la fig.5, la concavité des cavités 15 et 15' de l'élément intermédiaire 3, prise dans le plan sagittal latéralisé, est rigoureusement complémentaire à la convexité des condyles 16 et 16' de l'élément fémoral 2.

Le rayon R1 de courbure de ces condyles 16,16' et cavités 15,15' dans le plan sagittal, et donc de guidage de l'élément fémoral 2 en rotation sur l'élément intermédiaire 3, est constant et est de l'ordre compris entre 20 mm et 30 mm selon la taille de la prothèse, sur une plage P1 de surface de guidage de l'ordre de 155° de l'élément fémoral, correspondante à sa zone postérieure.

En revenant à la fig.2, on remarquera que l'axe a de ce rayon R1 de guidage est placé à l'arrière de la prothèse, dans le plan sagittal, par rapport à l'axe A d'extension de la quille 12 de l'élément intermédiaire 3, et donc de l'axe de pivot sur lui-même de l'élément intermédiaire 3 sur l'élément tibial 1. Ce décalage vers l'arrière de l'axe a est d'une distance d1 comprise entre 7 mm et 11 mm selon la taille de la prothèse. On remarquera aussi sur cette même figure que, dans le plan sagittal, cet axe A de pivot est décalé vers la zone antérieure de l'élément intermédiaire 3 par rapport à l'axe médian du plateau 4 de l'élément tibial 1, d'une distance d3 de l'ordre 0,035 fois la distance d4, soit d'une distance d3 comprise entre 1,5 mm et 2,2 mm selon la taille de la prothèse.

Sur les fig.3 à fig.5 plus particulièrement, la zone postérieure de guidage de l'élément fémoral 2 est prolongée par une zone médiane 19 courbe à grand rayon de courbure dans le plan sagittal, cette zone médiane 19 étant ensuite fortement recourbée pour former la zone antérieure 11 de l'élément fémoral 2.

Sur les fig.6 à fig.8, l'élément intermédiaire 3 est rehaussé dans le prolongement des cavités 15 et 15' d'une lèvre 20 formée de renflements ménagés de part et d'autre du bossage 17 de l'élément intermédiaire 3, et donc de part et d'autre de l'échancrure de l'élément fémoral 2 lorsque ce dernier est en appui contre l'élément intermédiaire 3. On notera que le rayon de courbure R2 du bossage 17 de l'élément intermédiaire 3, et donc celui correspondant de l'échancrure de l'élément fémoral 2, est de l'ordre de 10 mm pris dans le plan frontal. Le bord supérieur de la lèvre 20 est disposé à une distance d2 du fond des cavités 15,15' de l'élément intermédiaire 3, de l'ordre comprise entre 13 mm et 18 mm selon la taille de la prothèse.

En revenant à la fig.4, on remarquera la présence souhaitée de dégagements 21 ménagés à la face externe dans la zone médiane 19 de l'élément fémoral 2, pour la réception des bords supérieurs correspondants des lèvres 20.

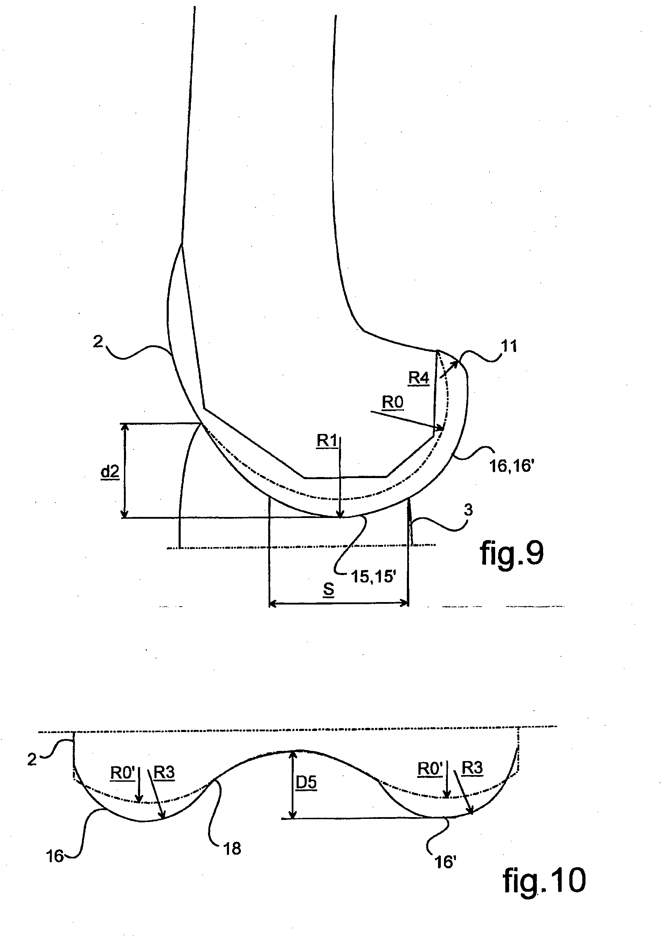

Sur la fig.9, l'élément fémoral 2 d'une prothèse du genou de l'invention présente un profil des condyles 16,16' dont le rayon R1 est supérieur au rayon correspondant R0 des condyles naturels d'un fémur, et s'étend sur une plage de 150° au moins. Selon l'art antérieur, un tel rayon R0 est au plus correspondant au rayon naturel des condyles d'un fémur, et s'étend sur une plage de l'ordre de 120° au plus. Un tel rayon R1 selon la présente invention permet d'augmenter la distance d2 antéropostérieure entre le bord antérieur 20 de l'élément intermédiaire 3, et la surface des condyles 16,16' de l'élément fémoral 2 circulant en fond des cavités 15,15' de l'élément intermédiaire 3, et permet de préserver le capital osseux du patient. Une telle augmentation de la distance d2 permet l'augmentation des surfaces d'appui S entre l'élément fémoral 2 et l'élément intermédiaire 3, et surtout un recentrage vers l'avant des charges, sans changer l'axe de rotation interepicondylien et sans perte du moment d'extension. Au-delà de la plage de rotation définie par le rayon R1, le rayon R4 se réduit significativement pour ménager la zone 11 recourbée de l'élément fémoral 2.

Sur la fig.10, le rayon R3 des condyles 16,16' de l'élément fémoral 2, correspondant à celui de la concavité des cavités 15 et 15' de l'élément intermédiaire 3, est lui aussi supérieur à celui du rayon naturel R0' correspondant des condyles d'un fémur, un tel rayon R0' étant habituellement mis en oeuvre dans les prothèses de l'art antérieur. Ces dispositions de l'invention permettent d'optimiser la congruence grâce à une optimisation de la distance D5 du mouvement rotatoire séparant le fond de l'échancrure 18 de l'élément fémoral 2 de l'extrémité des condyles 16,16' de ce dernier.

On notera que les dispositions prévues par la présente invention sont notamment applicables à une prothèse totale du genou en raison des avantages procurés, mais sont néanmoins transposables à des prothèses de demi genou ou unicompartimentale par reproduction partielle latérale pour le guidage de l'un ou l'autre des condyles de l'élément fémoral le long d'une cavité correspondante de l'élément intermédiaire, voire d'un élément tibial.

## Revendications

1. Prothèse totale du genou, du genre de prothèse composée :
*) d'un élément tibial (1) à plateau (4), qui est pourvu de moyens d'ancrage et de centrage (5) à l'extrémité d'un tibia et de moyens de réception d'un épaulement (13) et d'une quille (12) d'emboîtement axial et de centrage d'un élément intermédiaire (3),
*) d'un élément fémoral (2) pourvu de moyens d'ancrage et de centrage (6) à l'extrémité d'un fémur, qui sont ménagés sur une face interne de conformation globalement complémentaire à celle des condyles et de l'échancrure naturels du fémur préalablement préparés, pour les envelopper entre deux facettes opposées (10,10') distantes l'une de l'autre d'une distance d4, tandis qu'une face externe de l'élément fémoral comporte un couple de condyles (16,16') séparés par une échancrure (18), qui sont ménagés sur la partie postérieure de l'élément fémoral (2) pour former une surface de guidage de ce dernier sur l'élément intermédiaire (3), le profil de ces condyles (16,16') s'étendant suivant un rayon R1 constant de rotation,
*) ledit élément intermédiaire (3), qui est interposé entre l'élément tibial (1) et l'élément fémoral (2) pour autoriser une mobilité de l'élément fémoral (2) sur l'élément intermédiaire (3), en rotation dans le plan sagittal autour d'un axe horizontal a, en basculement d'un condyle (16,16') à l'autre, cet élément intermédiaire (3) comportant à sa face externe un logement composé de cavités (15,15') séparées l'une de l'autre par un bossage (17), pour la réception respectivement des condyles (16,16') et de l'échancrure (18) de l'élément fémoral,
**caractérisée en ce que**,
- ledit élément intermédiaire (3) autorise un pivotement axial de l'élément intermédiaire (3) sur l'élément tibial (1) et
- pris dans le plan sagittal latéralisé relatif à l'un et/ou l'autre des condyles (16,16') de l'élément fémoral (2), ledit rayon R1 constant de rotation du profil des condyles (16,16') est de l'ordre de 0,5 fois la distance d4 de séparation desdites facettes opposées (10,10') de l'élément fémoral (2), pondérée de plus ou moins 15% selon la taille de la prothèse, pour optimiser les surfaces de contact en préservant le capital osseux du fémur, ledit rayon constant s'étendant sur une plage P1 de surface de guidage angulaire correspondante de l'ordre de 150° au moins.

2. Prothèse totale du genou selon la revendication 1, caractérisée en ce l'axe a de rotation des condyles (16,16') sur l'élément intermédiaire (3) est décalé dans le plan sagittal vers l'arrière d'une distance d1 par rapport à l'axe A de pivotement de l'élément intermédiaire (3) sur l'élément tibial (1), de sorte que le moment d'action en extension de la prothèse soit important, et que les possibilités de flexion soient augmentées en empêchant un contact prématuré entre la face postérieure du fémur et le bord postérieur de l'élément tibial (1).

3. Prothèse totale du genou selon la revendication 2, **caractérisée en ce que** ladite distance d1 est de l'ordre de 0,18 fois la distance d4 de séparation desdites facettes opposées (10,10') de l'élément fémoral (2), pondérée de plus ou moins 10% selon la taille de la prothèse.

4. Prothèse totale du genou selon l'une quelconque des revendications précédentes, **caractérisée en ce que** le bord antérieur de l'élément intermédiaire (3) étant rehaussé d'une lèvre (20), celle-ci est ménagée en prolongement de l'une et l'autre des cavités (15,15') de l'élément intermédiaire (3).

5. Prothèse totale du genou selon la revendication 4, **caractérisée en ce que** la distance d2 de séparation entre le fond des cavités (15,15') et le bord supérieur de la lèvre (20) est de l'ordre de 0,32 fois la distance d4 de séparation desdites facettes opposées (10,10') de l'élément fémoral (2), pondérée de plus ou moins 5% selon la taille de la prothèse.

6. Prothèse totale du genou selon l'une quelconque des revendications précédentes, **caractérisée en ce que** le débattement angulaire de l'élément fémoral (2) sur l'élément intermédiaire (3) entre les stations extrêmes d'extension et de flexion est de l'ordre de 120°, avec des surfaces de portée sur une plage de surface angulaire de l'ordre de 90° qui est sensiblement constante depuis la station d'extension à 0° de verticalité jusqu'à 90° de flexion, puis avec une surface de portée se réduisant fortement jusqu'à 120° de flexion.

7. Prothèse totale du genou selon l'une quelconque des revendications précédentes, **caractérisée en ce que**, pris dans le plan frontal, le rayon R3 des cavités (15,15') de l'élément intermédiaire (3) est de l'ordre de 0,6 fois la distance d4 de séparation desdites facettes opposées (10,10') de l'élément fémoral (2), le rayon R2 du bossage (17) de l'élément intermédiaire (3) étant quant à lui de l'ordre de 10 mm.

8. Prothèse totale du genou selon l'une quelconque des revendications précédentes, **caractérisée en ce que** la face interne de l'élément fémoral (2) comporte, disposées de part et d'autre d'un bossage (7) qui correspond à la face opposée de l'échancrure (18) de l'élément fémoral (2) et qui est destiné à coopérer avec l'échancrure du fémur, des cavités de réception respective des condyles droit et gauche du fémur, qui sont chacune conformées en un plan médian (8) comportant au moins un picot (6) d'ancrage de l'élément fémoral (2) dans le fémur, ce plan médian (8) étant bordé de part et d'autre par des plans inclinés à 45° respectivement antérieur (9) et postérieur (9'), ces plans inclinés (9,9') étant respectivement bordés par des plans d'extrémité antérieur (10) et postérieur (10') formant lesdites facettes opposées, ces plans d'extrémité antérieur (10) et postérieur (10') délimitant ensemble un angle ouvert.

9. Prothèse totale du genou selon l'une quelconque des revendications précédentes, **caractérisée en ce que** la partie postérieure de l'élément fémoral (2), prise dans le plan sagittal latéralisé relatif à l'un et/ou l'autre des condyles (16,16'), est prolongée par une partie antérieure (19) de l'élément fémoral (2) à large rayon de courbure, lui conférant un profil sensiblement droit, qui est ensuite fortement recourbée (11).

## Patentansprüche

1. Knievollprothese der Bauart einer Prothese, die sich aus Folgendem zusammensetzt:
*) einem schienbeinseitigen Element (1) das eine Platte (4) aufweist und das mit Mitteln (5) zum Verankern und Zentrieren am Ende eines Schienbeins versehen ist sowie mit Mitteln zur Aufnahme eines Ansatzes (13) eines Zapfens (12), welcher zum axialen Aufstecken und Zentrieren eines Zwischenelements (3) bestimmt ist,
*) einem oberschenkelknochenseitigen Element (2), das mit Mitteln (6) zum Verankern und Zentrieren am Ende eines Oberschenkelknochens versehen ist, wobei diese auf einer Innenseite angeordnet sind, deren Formgebung insgesamt zu den im Vorfeld vorbereiteten natürlichen Gelenkknorren und der Ausnehmung des Oberschenkelknochens komplementär ist, um diese zwischen zwei Seiten (10, 10') zu umhüllen, welche sich in einem Abstand d4 gegenüberliegen, wohingegen eine Außenseite des oberschenkelknochenseitigen Elements ein Paar von Gelenkknorren (16, 16') aufweist, die durch eine Ausnehmung (18) getrennt sind, wobei sie am hinteren Ende des oberschenkelknochenseitigen Elements (2) angeordnet sind, um eine Fläche zu bilden, die zur Führung des letzteren auf dem Zwischenelement (3) dient, wobei sich das Profil dieser Gelenkknorren (16, 16') gemäß einem konstanten Drehradius R1 erstreckt.
*) dem Zwischenelement (3), welches zwischen dem schienbeinseitigen Element (1) und dem oberschenkelknochenseitigen Element (2) angeordnet ist, um es zu ermöglichen, dass das oberschenkelknochenseitige Element (2) auf dem Zwischenelement (3) innerhalb der Sagittalebene eine Drehbewegung um eine horizontale Achse ausführen kann, indem es zu einem Kippvorgang von einem Gelenkknorren (16, 16') zum anderen kommt, wobei das Zwischenelement (3) auf seiner Außenseite eine Aufnahme aufweist, die aus Hohlräumen (15, 15') besteht, welche durch einen Höcker (17) voneinander getrennt sind, um die Gelenkknorren (16, 16') beziehungsweise die Ausnehmung (18) des oberschenkelknochenseitigen Elements aufzunehmen,
**dadurch gekennzeichnet, dass**:
- das Zwischenelement (3) eine axiale Schwenkbewegung des Zwischenelements (3) auf den schienbeinseitigen Element (1) ermöglicht, und
- bei einer Betrachtung in der Sagittalebene, die unter Bezugnahme auf den einen und/oder den anderen der Gelenkknorren (16, 16') des oberschenkelknochenseitigen Elements (2) seitlich gelegen ist, der konstante Drehradius R1 des Profils der Gelenkknorren (16, 16') eine Größenordnung hat, welche 0,5-mal dem Abstand d4 zwischen den gegenüberliegenden Seiten (10, 10') des oberschenkelknochenseitigen Elements (2) entspricht, wobei eine Gewichtung von plus oder minus 15 % je nach der Größe der Prothese erfolgt, um die Kontaktflächen zwecks Erhaltung des Oberschenkelknochenkapitals zu optimieren, wobei der konstante Radius sich über einen entsprechenden Bereich P1 der Führungsfläche erstreckt, dessen Winkelweite einer Größenordnung von mindestens 150° entspricht.

2. Knievollprothese nach Anspruch 1, **dadurch gekennzeichnet, dass** die Achse a, gemäß derer sich die Gelenkknorren (16, 16') auf dem Zwischenelement (3) drehen, innerhalb der Sagittalebene gegenüber der Achse A, gemäß derer das Zwischenelement (3) auf dem schienbeinseitigen Element (1) schwenkt, um einen Abstand d1 nach hinten versetzt ist, dass die Prothese ein hohes Dehnwirkungsmoment aufweist und dass die Beugemöglichkeiten dadurch erhöht sind, dass ein vorzeitiger Kontakt zwischen der Hinterseite des Oberschenkelknochens und dem hinteren Rand des schienbeinseitigen Elements (1) vermieden wird.

3. Knievollprothese nach Anspruch 2, **dadurch gekennzeichnet, dass** die Größenordnung des Abstands d1 derart beschaffen ist, dass sie dem 0,18-fachen des Abstandes d4 entspricht, welcher die gegenüberliegenden Seiten (10, 10') des oberschenkelknochenseitigen Elements (2) voneinander trennt, wobei eine Gewichtung von plus oder minus 10 % je nach der Größe der Prothese erfolgt.

4. Knievollprothese nach einem beliebigen der vorhergehenden Ansprüche, dadurch gekenntzeichnet, dass auf den hinteren Rand des Zwischenelements (3) eine Lippe (20) aufgesetzt ist, die derart angeordnet ist, dass sie einen der beiden Hohlräume (15, 15') des Zwischenelements (3) verlängert.

5. Knievollprothese nach Anspruch 4, **dadurch gekennzeichnet, dass** die Größenordnung des Abstands d2, welcher den Boden der Hohlräume (15, 15') vom oberen Rand der Lippe (20) trennt, derart beschaffen ist, dass sie dem 0,32-fachen des Abstandes d4 entspricht, welcher die gegenüberliegenden Seiten (10, 10') des oberschenkelknochenseitigen Elements (2) voneinander trennt, wobei eine Gewichtung von plus oder minus 5 % je nach der Größe der Prothese erfolgt.

6. Knievollprothese nach einem beliebigen der vorhergehenden Ansprüche, **dadurch gekennzeichnet, dass** der Winkelausschlag des oberschenkelknochenseitigen Elements (2) auf dem Zwischenelement (3), zwischen den äußersten Dehn- und Beugestellungen, in der Größenordnung von 120 ° liegt, wobei die Auflageflächen sich auf einem Flächenbereich befinden, dessen Winkelweite in der Größenordnung von 90° liegt und von der Dehnungsstellung mit einer Vertikalität von 0° bis zu einer Beugung von 90° im Wesentlichen konstant ist, woraufhin sich eine Auflagefläche stark verringert, wenn die Beugung auf bis zu 120° erhöht wird.

7. Knievollprothese nach einem beliebigen der vorhergehenden Ansprüche, **dadurch gekennzeichnet, dass** bei einer Betrachtung in der Frontalebene die Größenordnung des Radius R3 der Hohlräume (15, 15') des Zwischenelements (3) derart beschaffen ist, dass sie dem 0,6-fachen des Abstands d4 entspricht, welcher die gegenüberliegenden Seiten (10, 10') des oberschenkelknochenseitigen Elements (2) voneinander trennt, wobei der Radius R2 des Höckers (17) des Zwischenelements (3) wiederum in der Größenordnung von 10 mm liegt.

8. Knievollprothese nach einem beliebigen der vorhergehenden Ansprüche, **dadurch gekennzeichnet, dass** die Innenseite des oberschenkelknochenseitigen Elements (2) mit Hohlräumen zur Aufnahme des linken beziehungsweise rechten Gelenkknorrens des Oberschenkelknochens versehen ist, wobei diese Hohlräume beiderseits eines Höckers (7) angeordnet sind, welcher der gegenüberliegenden Seite der Ausnehmung (18) des oberschenkelknochenseitigen Elements (2) entspricht und welcher dazu bestimmt ist, mit der Ausnehmung des Oberschenkelknochens zusammenzuwirken, wobei jeder der Hohlräume einer Medianebene (8) derart ausgestaltet ist, dass er mindestens einen Stift (6) zur Verankerung des oberschenkelknochenseitigen Elements (2) im Oberschenkelknochen aufweist, wobei an diese Medianebene (8) beiderseits eine vorderen (9) beziehungsweise hintere (9') um 45 ° geneigte Fläche grenzt; wobei an die geneigten Flächen (9, 9') jeweils eine vordere (10) und eine hintere (10') endständige Fläche grenzt, welche die gegenüberliegenden Flächen bilden, wobei die vordere (10) und die hintere (10') endständige Fläche gemeinsam einen offenen Winkel begrenzen.

9. Knievollprothese nach einem beliebigen der vorhergehenden Ansprüche, **dadurch gekennzeichnet, dass** der hintere Abschnitt des oberschenkelknochenseitigen Elements (2), bei einer Betrachtung in der Sagittalebene, die unter Bezugnahme auf den einen und/oder den anderen der Gelenkknorren (16, 16') seitlich gelegen ist, von einem vorderen Abschnitt (19) des oberschenkelknochenseitigen Elements (2) mit großem Krümmungsradius verlängert wird, sodass ihm ein im Wesentlichen geradliniges Profil verliehen wird, wobei er sich im weiteren Verlauf stark krümmt (11).

## Claims

1. Total knee replacement prosthesis, of the type of prosthesis comprised of:
*) a tibial element (1) with tray (4), which is provided with means of anchoring and centering (5) at the end of a tibia and means of receiving a shoulder (13) and an axial interlocking and centering fin (12) of an intermediate element (3),
*) a femoral element (2) equipped with means of anchoring and centering (6) at the end of a femur, which are formed on an internal conforming surface mainly complementary to that of the previously prepared natural condyles and indentation of the femur, to envelop them between two opposite facets (10,10') separated from each other by a distance d4, while an external surface of the femoral element comprises a pair of condyles (16,16') separated by an indentation (18), which are formed on the posterior portion of the femoral element (2) to form a guide surface for the latter on the intermediate element (3), the profile of these condyles (16,16') extends along a constant radius R1 of rotation,
*) said intermediate element (3), which is interposed between the tibial element (1) and the femoral element (2) to permit mobility of the femoral element (2) on the intermediate element (3), in rotation in the sagittal plane around a horizontal axis a, tilting from one condyle (16,16') to the other, this intermediate element (3) comprising on its external surface a recess consisting of cavities (15,15') separated from each other by a projection (17), for receiving respectively the femoral element's condyles (16,16') and indentation (18),
**characterized in that**,
- said intermediate element (3) permits the intermediate element (3) to pivot axially on the tibial element (1) and
- taken in the lateralized sagittal plane relative to either of the condyles (16,16') of the femoral element (2), said constant radius R1 of rotation of the profile of the condyles (16,16') is of the order of 0.5 times the distance d4 separating said opposite facets (10,10') of the femoral element (2), weighted by plus or minus 15% depending on the size of the prosthesis, so as to optimize the contact surfaces while preserving the femur's bone stock, said constant radius extending over a corresponding angular guide surface range P1 of at least 150°.

2. Total knee replacement prosthesis according to claim 1, **characterized in that** the rotation axis a of the condyles (16,16') on the intermediate element (3) is offset in the sagittal plane towards the rear by a distance d1 relative to the pivot axis A of the intermediate element (3) on the tibial element (1), such that the action moment in extension of the prosthesis is large and the possibilities of flexion are increased by preventing premature contact between the posterior surface of the femur and the posterior edge of the tibial element (1).

3. Total knee replacement prosthesis according to claim 2, **characterized in that** said distance d1 is of the order of 0.18 times the distance d4 separating said opposite facets (10,10') of the femoral element (2), weighted by plus or minus 10% depending on the size of the prosthesis.

4. Total knee replacement prosthesis according to any one of the preceding claims, **characterized in that** the anterior edge of the intermediate element (3) being enhanced by a lip (20), the latter is formed in extension of either one of the cavities (15,15') of the intermediate element (3).

5. Total knee replacement prosthesis according to claim 4, **characterized in that** the separation distance d2 between the bottom of the cavities (15,15') and the upper edge of the lip (20) is of the order of 0.32 times the distance d4 separating said opposite facets (10,10') of the femoral element (2), weighted by plus or minus 5% depending on the size of the prosthesis.

6. Total knee replacement prosthesis according to any one of the preceding claims, **characterized in that** the angular travel of the femoral element (2) on the intermediate element (3) between the extreme extension and flexion positions is of the order of 120°, with bearing surfaces over an angular surface range of the order of 90° which is substantially constant from the extension position at 0° of verticality up to 90° of flexion, then with a bearing surface reducing significantly up to 120° of flexion.

7. Total knee replacement prosthesis according to any one of the preceding claims, **characterized in that**, taken in the frontal plane, the radius R3 of the cavities (15,15') of the intermediate element (3) is of the order of 0.6 times the distance d4 separating said opposite facets (10,10') of the femoral element (2), the radius R2 of the projection (17) of the intermediate element (3) being of the order of 10 mm.

8. Total knee replacement prosthesis according to any one of the preceding claims, **characterized in that** the interior surface of the femoral element (2) comprises, arranged each side of a projection (7) that corresponds to the opposite surface of the indentation (18) of the femoral element (2) and which is designed to cooperate with the indentation of the femur, reception cavities respectively of the right and left condyles of the femur, each of which is shaped in a median plane (8) comprising at least one pin (6) for anchoring the femoral element (2) in the femur, this median plane (8) being edged on each side by respectively anterior (9) and posterior (9') planes inclined at 45°, these inclined planes (9,9') being edged respectively by anterior (10) and posterior (10') end planes forming said opposites facets, these anterior (10) and posterior (10') end planes together delimiting an open angle.

9. Total knee replacement prosthesis according to any one of the preceding claims, **characterized in that** the posterior portion of the femoral element (2), taken in the lateralized sagittal plane relative to either of the condyles (16,16'), is prolonged by an anterior portion (19) of the femoral element (2) with a large radius of curvature, giving it a substantially straight profile, which is then significantly curved (11).
